# EUROPEAN PATENT APPLICATION

(11) **EP 3 170 831 A1**
(43) Date of publication of application: **24.05.2017**
(21) Application number: 16194174.5
(22) Date of filing: 06.09.2012
(51) Int. Cl.: C07H 1/08, C12Q 1/68, C12N 15/10

(54) **SAMPLE PREPARATION METHODS**

(30) Priority: 06.09.2011 US 201161531471 P
(62) Divisional of application: 12829439.4
(71) Applicant: Ibis Biosciences, Inc., Carlsbad, CA 92008 (US)
(72) Inventor: ESHOO, Mark, W., San Diego, CA 92130 (US); CROWDER, Christopher, Vista, CA 92081 (US)
(74) Representative: Chapman, Desmond Mark

(57) **Abstract**

The present invention provides whole blood nucleic acid extraction methods, compositions, and kits, as well as nested isothermal amplification methods, compositions, and kits. In certain embodiments, these methods are applied to detecting Lyme disease, including in patients without classic erythema migrans skin lesions.

## Description

### FIELD OF THE INVENTION

The present invention provides whole blood nucleic acid extraction methods, compositions, and kits, as well as nested isothermal amplification methods, compositions, and kits. In certain embodiments, these methods are applied to detecting Lyme disease, including in patients without classic erythema migrans skin lesions.

### BACKGROUND

The diagnosis of acute infectious diseases is problematic when the pathogens are not in abundance, not easily cultured or the antibody response to them is delayed. Lyme disease is such an example. Infection by the causative agent, *Borrelia burgdorferi^{1,2}*, is often difficult to diagnose because of clinical variability, including variations of the erythema migrans (EM) skin lesion and the lack of high performing diagnostic tests. A strategy that results in early unambiguous diagnosis of the infection will favorably impact management of the patient.

There are currently clinical limitations to early diagnosis. The best clinical marker of acute infection, the skin lesion erythema migrans (EM), is absent in at least 30% of the cases.³ Even when present, the EM is often not the classic bull's-eye lesion but rather an uncharacteristic variant as reported in 25-30% of cases.^{4,5} This may require referral to a specialist or prompt further diagnostic testing, thereby delaying the start of antibiotic therapy at the time for the best chance of cure without sequelae.

These clinical hurdles to diagnosis are compounded by the limitations of current laboratory tests. The most commonly used assays are serologic. However, these assays have been hampered by the biologically delayed antibody response (often 3 weeks or more to reach detectable levels). They are further limited because a *single* positive test is an indirect measure of exposure and cannot distinguish active from past infection. A direct *Borrelia* molecular test such as PCR can have high specificity but typically the tests have lacked sensitivity when applied to blood. In the past, PCR assays for Lyme disease did not have the benefit of current advances in sample preparation.⁶ and enhancement methods.^{7,8} such as *Borrelia*-targeted DNA enrichment (akin to a "molecular culture") that may allow detection of a low number of nucleic acid copies in the blood. In addition, the optimal blood volume and component of the sample, such as whole blood versus serum, may have contributed to prior limitations of PCR based diagnostics.

### SUMMARY OF THE INVENTION

The present invention provides whole blood nucleic acid extraction methods, compositions, and kits, as well as nested isothermal amplification methods, compositions, and kits. In certain embodiments, these methods are applied to detecting Lyme disease, including in patients without classic erythema migrans skin lesions.

In some embodiments, the present description provides methods of nucleic acid extraction comprising: a) contacting a sample of whole blood (e.g., EDTA treated whole blood) with beads, a proteinase, and an anionic surfactant (e.g., SDS) to generate a treated sample; b) homogenizing the treated sample to generate a cell lysate; c) centrifuging the cell lysate comprising a supernatant; d) separating the supernatant from the cell lysate; e) adding magnetic particles and lysis buffer to the supernatant to generate a magnetic-particle sample, wherein the magnetic particles are configured to bind nucleic acid molecules; f) washing the magnetic-particle sample with a wash buffer; g) treating the magnetic-particle sample in order to generate a dried magnetic bead sample; and h) treating the dried magnetic bead sample with an elution buffer such that a purified nucleic acid sample is generated that comprises purified nucleic acid.

In certain embodiments, the methods further comprise subjecting the purified nucleic acid to PCR and/or isothermal nested PCR to generate amplified nucleic acid. In some embodiments, the methods further comprise subjecting the amplified nucleic acid to mass spectrometry bioagent analysis in order to identify the source of the purified nucleic acid. In other embodiments, the mass spectrometry bioagent analysis comprises electrospray ionization mass spectrometry and base composition analysis.

In some embodiments, the present disclosure provides methods comprising: a) contacting a sample comprising isolated nucleic acid with a buffer, dNTPs, and a plurality of nested PCR primer pairs configured to amplify at least part of at least one bioagent target sequence; b) incubating the sample with a DNA polymerase under isothermal conditions such that amplified nucleic acid is generated; c) inactivating the DNA polymerase; and d) subjecting the amplified nucleic acid to mass spectrometry bioagent analysis in order to identify the source of the isolated nucleic acid.

In other embodiments, the mass spectrometry bioagent analysis comprises electrospray ionization mass spectrometry and base composition analysis. In certain embodiments, the DNA polymerase comprises BstE DNA polymerase. In additional embodiments, the incubating is conducted at about 56 degrees Celsius. In further embodiments, inactivating the DNA polymerase comprises heating the sample to at least about 80 degrees Celsius. In other embodiments, the plurality of nested PCR primer pairs comprises at least 10 primer pairs (e.g., 10 ... 15 ... 19, etc). In particular embodiments, the plurality of nested PCR primer pairs comprises at least 20 primer pairs (e.g., 20 ... 25 ... 35 ... 45 ... etc.). In further embodiments, the at least one bioagent target sequence comprises at least five bioagent target sequences. In certain embodiments, the methods further comprise a step after c) but before d) of further amplifying the amplified nucleic acid without any purification of the amplified nucleic acid.

### DESCRIPTION OF THE FIGURES

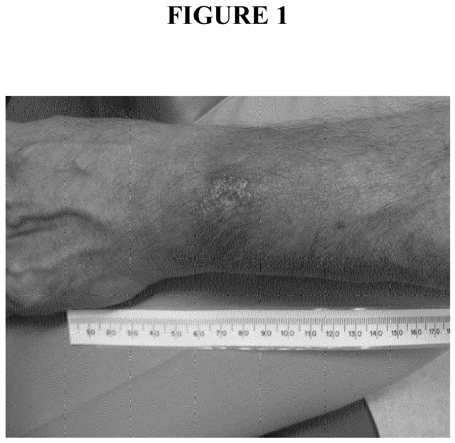

Figure 1 shows an atypical EM lesion from a patient who was PCR positive and seronegative with a negative ELISA after eight days of illness. Repeat serology at the end of therapy 3 weeks later showed a positive ELISA, positive IgM western blot and negative IgG western blot.

### DETAILED DESCRIPTION

The present invention provides whole blood nucleic acid extraction methods, compositions, and kits, as well as nested isothermal amplification methods, compositions, and kits. In certain embodiments, these methods are applied to detecting Lyme disease, including in patients without classic erythema migrans skin lesions.

The methods, kits, and compositions are useful with any target nucleic acid sequence and are not limited to any particular target sequence (e.g., not limited to nucleic acid sequences from *B. burgdorferi*). The discussion below is focused on detecting sequence from *B. burgdorferi.* Such target sequences are exemplary and are not intended to limit the scope of the present description. Other target sequences (e.g., from infections disease) are well known in the art. Also well know in the art are primers, including nested prior sets, that are useful in the present description.

Lyme disease is representative of an infectious disease where early diagnosis is imperative to avoid sequelae. However diagnosis is often difficult because the clinical manifestations, including the rash, are variable and the pathogens are often not in abundance, not easily cultured, and the antibody response to them is delayed. In the past PCR for *B. burgdorferi* in blood had low sensitivity. This may be related to low copy number, insufficient volume of blood or targeting the wrong component of blood

The present description overcomes some of these obstacles by combining a pre-PCR nucleic acid enrichment with sensitive PCR detection from nucleic acid extraction from 1.25 ml of whole blood from patients with skin lesions and early Lyme disease. With this strategy, PCR positivity was found in 14 of 29 (p=0·0001) endemic area subjects with typical erythema migrans (EM) or a variant, often before seroconversion was positive. The enrichment technique increased the yield from 2 to 14. None of 44 control subjects were positive. A serendipitous and unexpected finding of clinical importance was the observation that 8 of 14 (57%) of PCR positive subjects had an atypical EM.

The description provides improved early diagnosis of Lyme disease by combination of a pre-PCR Borrelia DNA enhancement, sensitive PCR, and targeting sufficient volumes of whole blood. The surprise finding of non-classic EM lesions in the majority of microbiologically proven Lyme disease cases serves as alert to clinicians evaluating patients with endemic area exposure.

In certain embodiments, the PCR generated amplicons are detected by mass spectrometry methods using bioagent identifying amplicons. In some embodiments, primers are selected to hybridize to conserved sequence regions of nucleic acids derived from a bioagent and which flank variable sequence regions to yield a bioagent identifying amplicon which can be amplified and which is amenable to base composition analysis. In some embodiments, the corresponding base composition of one or more different amplicons is queried against a database of base compositions indexed to bioagents and to the primer pair used to generate the amplicon. A match of the measured base composition to a database entry base composition associates the sample bioagent to an indexed bioagent in the database. Thus, the identity of the unknown bioagent is determined. No prior knowledge of the unknown bioagent is necessary to make an identification. In some instances, the measured base composition associates with more than one database entry base composition. Thus, a second/subsequent primer pair is generally used to generate an amplicon, and its measured base composition is similarly compared to the database to determine its identity in triangulation identification. Furthermore, the methods and other aspects of the invention can be applied to rapid parallel multiplex analyses, the results of which can be employed in a triangulation identification strategy. Thus, in some embodiments, the present invention provides rapid throughput and does not require nucleic acid sequencing or knowledge of the linear sequences of nucleobases of the amplified target sequence for bioagent detection and identification.

Methods of employing base compositions, databases containing base composition entries, and triangulation using primers, are described in the following patents, patent applications and scientific publications, all of which are herein incorporated by reference as if fully set forth herein: US patent numbers 7,108,974; 7,217,510; 7,226,739; 7,255,992; 7,312,036; 7,339,051; US patent publication numbers 2003/0027135; 2003/0167133; 2003/0167134; 2003/0175695; 2003/0175696; 2003/0175697; 2003/0187588; 2003/0187593; 2003/0190605; 2003/0225529; 2003/0228571; 2004/0110169; 2004/0117129; 2004/0121309; 2004/0121310; 2004/0121311; 2004/0121312; 2004/0121313; 2004/0121314; 2004/0121315; 2004/0121329; 2004/0121335; 2004/0121340; 2004/0122598; 2004/0122857; 2004/0161770; 2004/0185438; 2004/0202997; 2004/0209260; 2004/0219517; 2004/0253583; 2004/0253619; 2005/0027459; 2005/0123952; 2005/0130196 2005/0142581; 2005/0164215; 2005/0266397; 2005/0270191; 2006/0014154; 2006/0121520; 2006/0205040; 2006/0240412; 2006/0259249; 2006/0275749; 2006/0275788; 2007/0087336; 2007/0087337; 2007/0087338 2007/0087339; 2007/0087340; 2007/0087341; 2007/0184434; 2007/0218467; 2007/0218467; 2007/0218489; 2007/0224614; 2007/0238116; 2007/0243544; 2007/0248969; WO2002/070664; WO2003/001976; WO2003/100035; WO2004/009849; WO2004/052175; WO2004/053076; WO2004/053141; WO2004/053164; WO2004/060278; WO2004/093644; WO 2004/101809; WO2004/111187; WO2005/023083; WO2005/023986; WO2005/024046; WO2005/033271; WO2005/036369; WO2005/086634; WO2005/089128; WO2005/091971; WO2005/092059; WO2005/094421; WO2005/098047; WO2005/116263; WO2005/117270; WO2006/019784; WO2006/034294; WO2006/071241; WO2006/094238; WO2006/116127; WO2006/135400; WO2007/014045; WO2007/047778; WO2007/086904; WO2007/100397; and WO2007/118222, all of which are herein incorporated by reference.

Exemplary base-count related methods and other aspects of use in the methods, systems, and other aspects of the invention are also described in, for example, Ecker et al., Ibis T5000: a universal biosensor approach for microbiology. Nat Rev Microbiol. 2008 Jun 3.; Ecker et al., The Microbial Rosetta Stone Database: A compilation of global and emerging infectious microorganisms and bioterrorist threat agents.; Ecker et al., The Ibis T5000 Universal Biosensor: An Automated Platform for Pathogen Identification and Strain Typing.; Ecker et al., The Microbial Rosetta Stone Database: A common structure for microbial biosecurity threat agents.; Ecker et al., Identification of Acinetobacter species and genotyping of Acinetobacter baumannii by multilocus PCR and mass spectrometry. J Clin Microbiol. 2006 Aug;44(8):2921-32.; Ecker et al., Rapid identification and strain-typing of respiratory pathogens for epidemic surveillance. Proc Natl Acad Sci U S A. 2005 May 31;102(22):8012-7. Epub 2005 May 23.; Wortmann et al., Genotypic evolution of Acinetobacter baumannii Strains in an outbreak associated with war trauma, Infect Control Hosp Epidemiol. 2008 Jun;29(6):553-555.; Hannis et al., High-resolution genotyping of Campylobacter species by use of PCR and high-throughput mass spectrometry. J Clin Microbiol. 2008 Apr;46(4): 1220-5.; Blyn et al., Rapid detection and molecular serotyping of adenovirus by use of PCR followed by electrospray ionization mass spectrometry. J Clin Microbiol. 2008 Feb;46(2):644-51.; Eshoo et al., Direct broad-range detection of alphaviruses in mosquito extracts, Virology. 2007 Nov 25;368(2):286-95.; Sampath et al., Global surveillance of emerging Influenza virus genotypes by mass spectrometry. PLoS ONE. 2007 May 30;2(5):e489.; Sampath et al., Rapid identification of emerging infectious agents using PCR and electrospray ionization mass spectrometry. Ann N Y Acad Sci. 2007 Apr;1102: 109-20.; Hujer et al., Analysis of antibiotic resistance genes in multidrug-resistant Acinetobacter sp. isolates from military and civilian patients treated at the Walter Reed Army Medical Center. Antimicrob Agents Chemother. 2006 Dec;50(12):4114-23.; Hall et al., Base composition analysis of human mitochondrial DNA using electrospray ionization mass spectrometry: a novel tool for the identification and differentiation of humans. Anal Biochem. 2005 Sep 1;344(1):53-69.; Sampath et al., Rapid identification of emerging pathogens: coronavirus. Emerg Infect Dis. 2005 Mar;11(3):373-9.; Jiang Y, Hofstadler SA. A highly efficient and automated method of purifying and desalting PCR products for analysis by electrospray ionization mass spectrometry; Jiang et al., Mitochondrial DNA mutation detection by electrospray mass spectrometry; Russell et al., Transmission dynamics and prospective environmental sampling of adenovirus in a military recruit setting; Hofstadler et al., Detection of microbial agents using broad-range PCR with detection by mass spectrometry: The TIGER concept. Chapter in; Hofstadler et al., Selective ion filtering by digital thresholding: A method to unwind complex ESI-mass spectra and eliminate signals from low molecular weight chemical noise.; Hofstadler et al., TIGER: The Universal Biosensor.; Van Ert et al., Mass spectrometry provides accurate characterization of two genetic marker types in Bacillus anthracis.; Sampath et al., Forum on Microbial Threats: Learning from SARS: Preparing for the Next Disease Outbreak -- Workshop Summary (ed. Knobler SE, Mahmoud A, Lemon S.) The National Academies Press, Washington, D.C. 2004. 181-185.

### EXAMPLES

### EXAMPLE 1

### Early Lyme patient specimens.

The current Example is part of a larger, ongoing longitudinal cohort study of early Lyme disease being conducted in a suburban community of a medium-sized, Northeast city since the summer of 2008. Adult patients with early, untreated Lyme disease are referred to a primary care physician with infectious disease training (JA) and provide written consent to participate.

Eligible patients are required to be treatment naïve, to have a documented rash diagnosed as EM at time of enrollment, and to have evidence of systemic infection; typically manifest as dissemination of the primary EM lesion or concurrent onset of new flu-like or other symptoms. Patients with a prior history of Lyme disease or symptom duration of their current illness of longer than 3 months are excluded. ²⁰. Forty four negative control specimens were obtained from Biomed Supply Inc. (Carlsbad, CA). Specimens were collected at a donation site in Pennsylvania from healthy donors screened by Biomed Supply Inc. A paired 7mL tube of EDTA treated whole blood and 5-12mL of serum was provided for each control patient.

### Serological and other analyses.

At the initial, pre-treatment study visit, self-report demographics, medical history information, complete blood counts (CBC), complete metabolic panels (CMP) and 2-tier antibody testing for *B. burgdorferi* were performed as part of the patient clinical evaluation. All serologic testing for both the patient specimens and the negative controls was performed through the same commercial laboratory, and results were interpreted according to CDC criteria ¹¹. Based on these criteria, approximate illness duration was documented and included in determining serostatus; patients with less than 4 weeks illness duration were positive based on either IgM or IgG positivity on western blot analysis, whereas patients with greater than 4 weeks illness duration were required to be IgG positive. Photos of EM rashes were reviewed by a panel of experienced clinicians without knowledge of serologic or PCR status. Skin lesions with classic bull's eye appearance with central clearing and peripheral erythema were classified as classic EM lesions. EM with uniform red or red-blue appearance which lacked central clearing were classified as non-classic EM lesions.

### DNA extraction from blood specimens:

A combination of bead-beating and magnetic bead isolation was used to extract nucleic acids from 1.25mL of whole EDTA blood. The blood was mixed in 2.0 mL screw-cap tubes (Sarstedt, Newton NC) filled with 1.35 g of 0.1-mm yttria-stabilized zirconium oxide beads (Glen Mills, Clifton, NJ), 25 µL proteinase K solution (Qiagen, Valencia, CA), 142 µL of 20% SDS solution (Ambion, Austin, TX) and 1uL of DNA extraction control (Abbott Molecular, Des Plains, IL). The mixture was then homogenized in a Precellys 24 tissue homogenizer (Bioamerica Inc., Miami, FL) at 6,200 rpm for 3 sets of 90 sec with 5 sec between sets. The homogenized lysates were then incubated at 56°C for 15 min and then centrifuged for 3 min at 16,000 g in a bench top microcentrifuge. To isolate nucleic acids 1 mL of the supernatant was transferred to a 24-well deep-well Kingfisher plate (Thermo Scientific, Waltham, MA) along with 1.1 mL of Abbott lysis buffer (Abbott Molecular), and 160 µL of magnetic particles (Abbott Molecular). The specimens were incubated for 16.5 minutes in the lysis buffer at 56°C. Specimens were then washed once in Wash buffer I (Abbott Molecular), and three times in Wash buffer II (Abbott Molecular), with 1 min incubation for each wash step. The magnetic beads were then dried for 3 min at 65°C, and nucleic acids were eluted into 250 µL of elution buffer (Abbott Molecular), by incubating the magnetic particles at 65°C for 3 min.

### Nested isothermal amplification of B. burgdorferi target DNA.

To increase sensitivity of *B. burgdorferi* detection by PCR/ESI-MS the seven target regions (eight primer pairs) of the previously described *Borrelia* genotyping assay ²¹ were enriched by a nested isothermal amplification. For each of the seven target regions were amplified using 50 oligonucleotide primers: 25 upstream and 25 downstream to the DNA region of interest (Table 1).

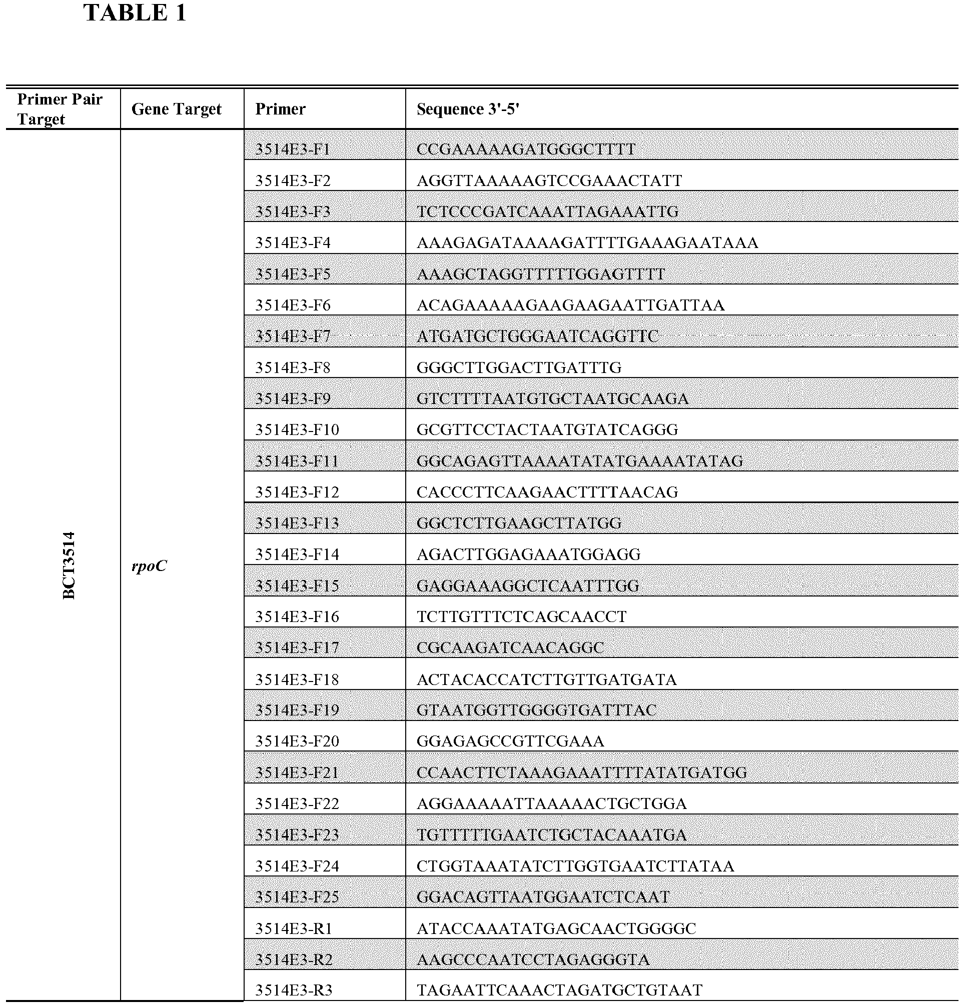

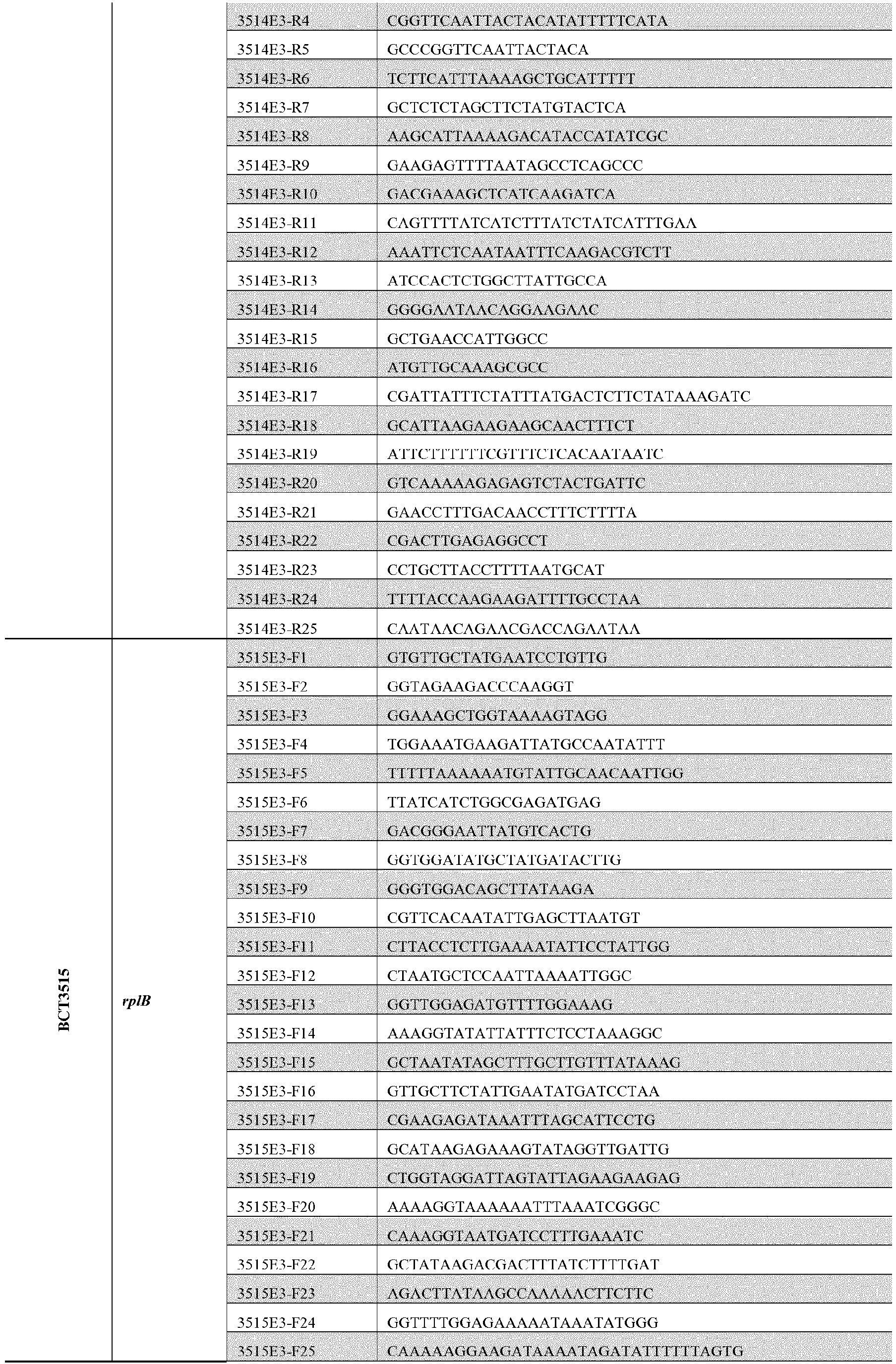

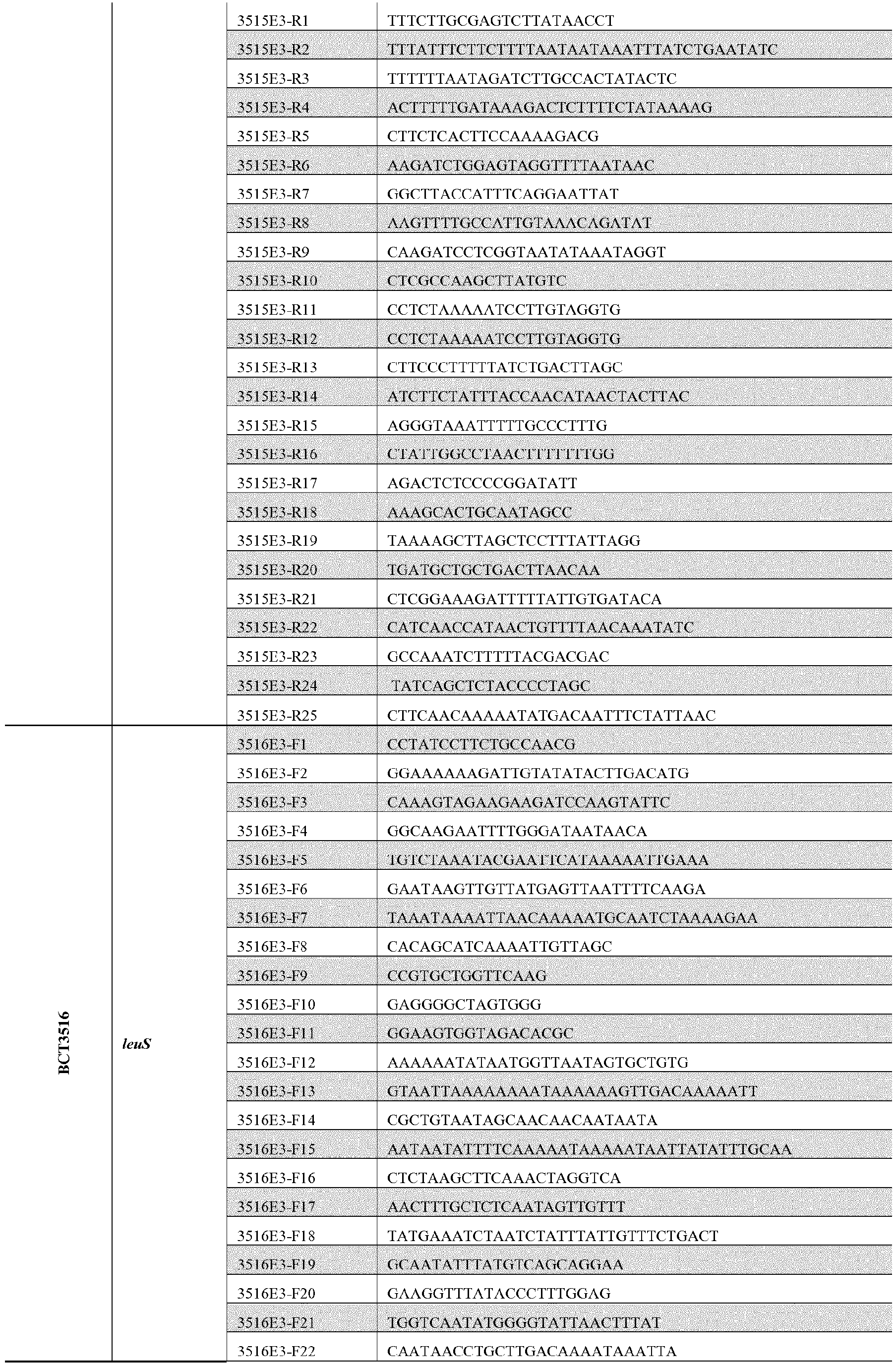

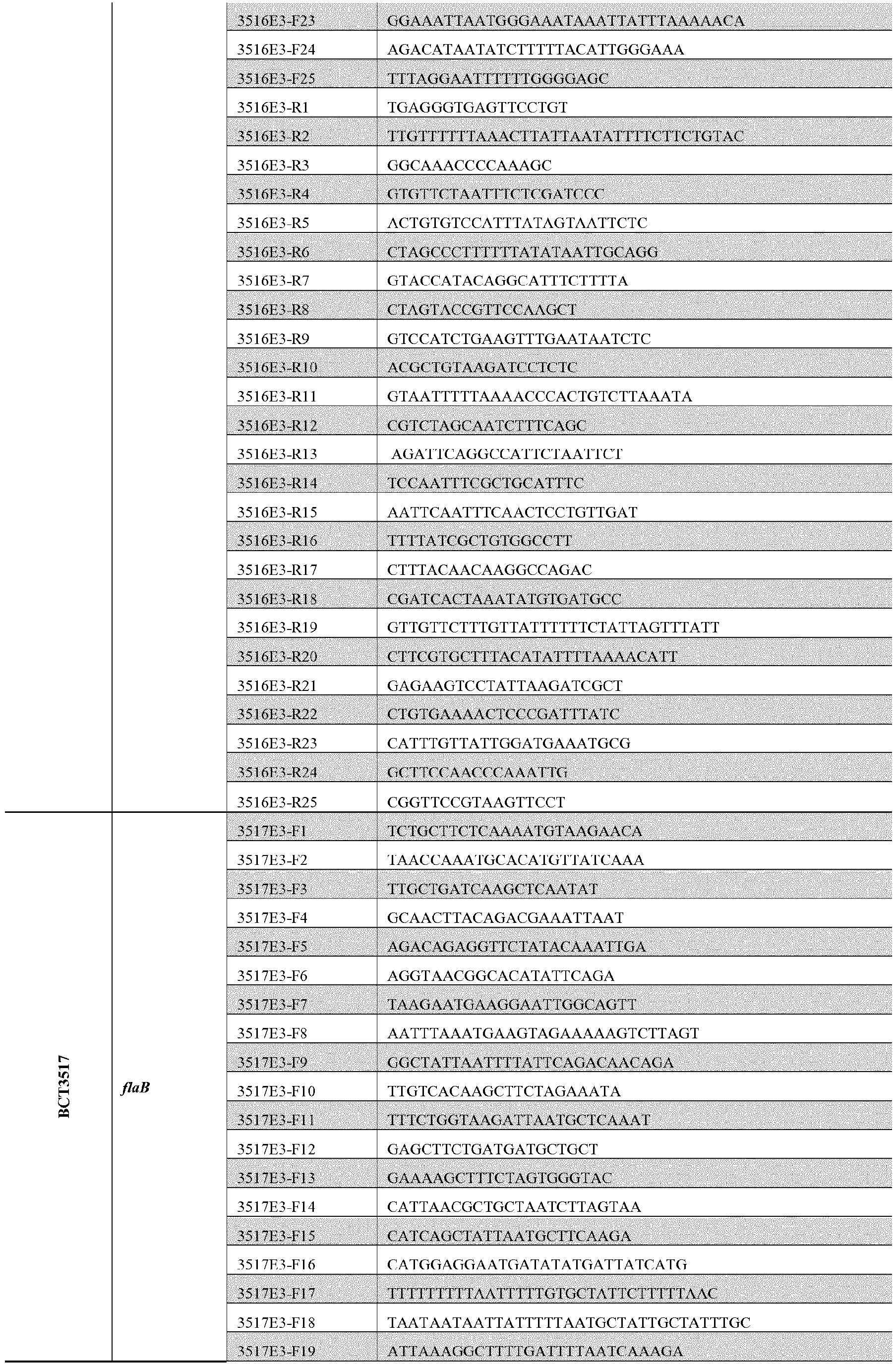

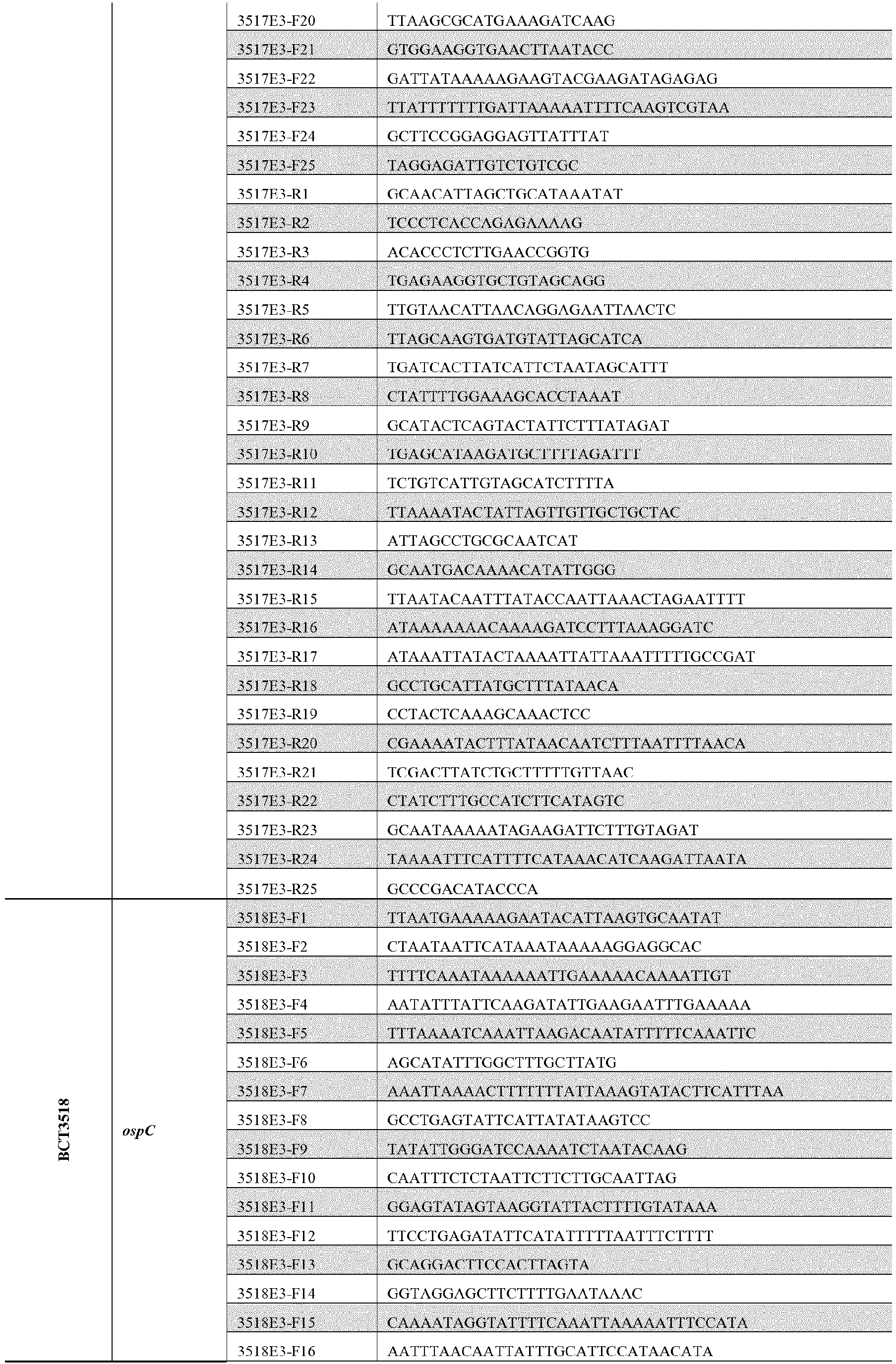

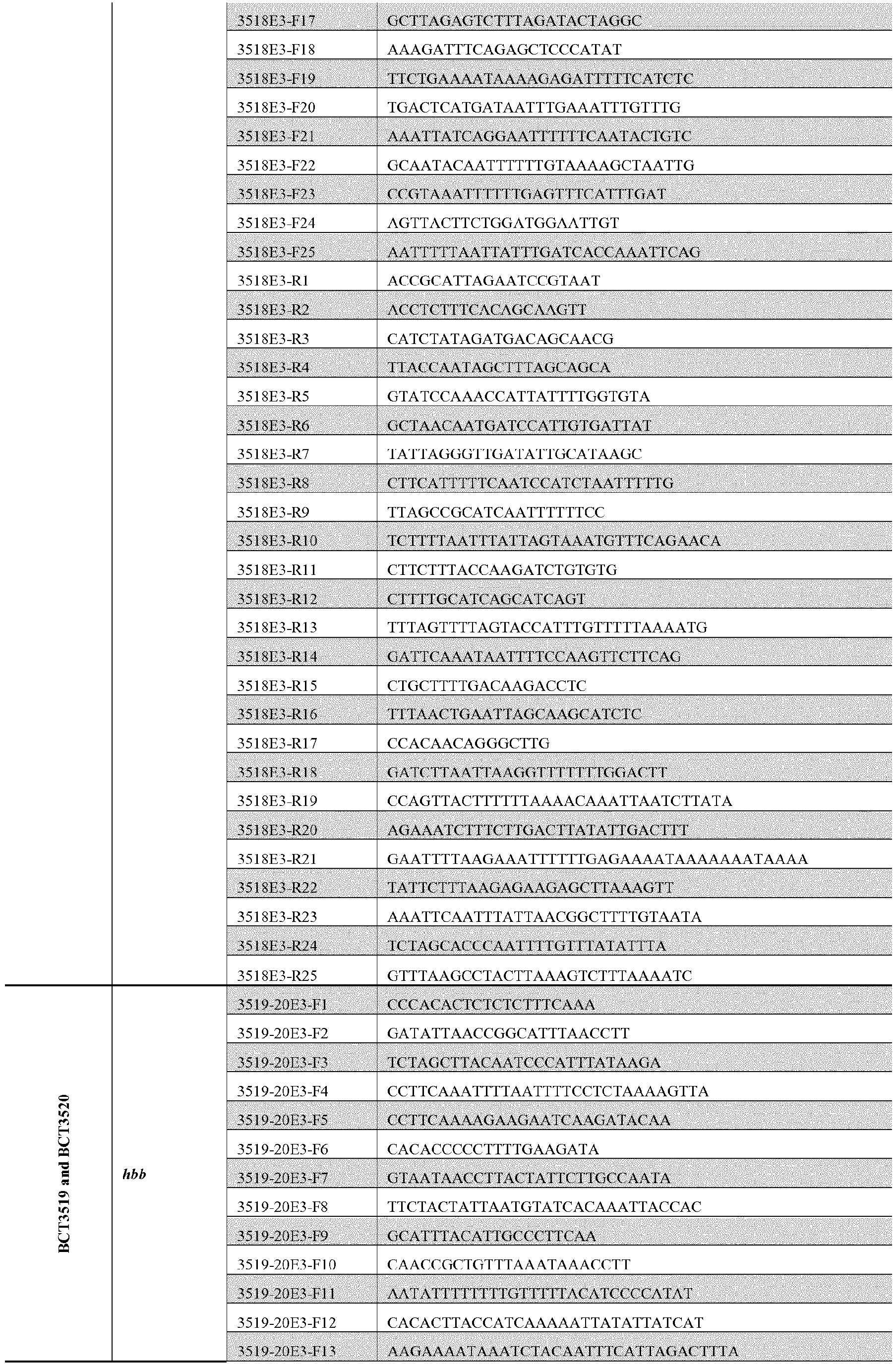

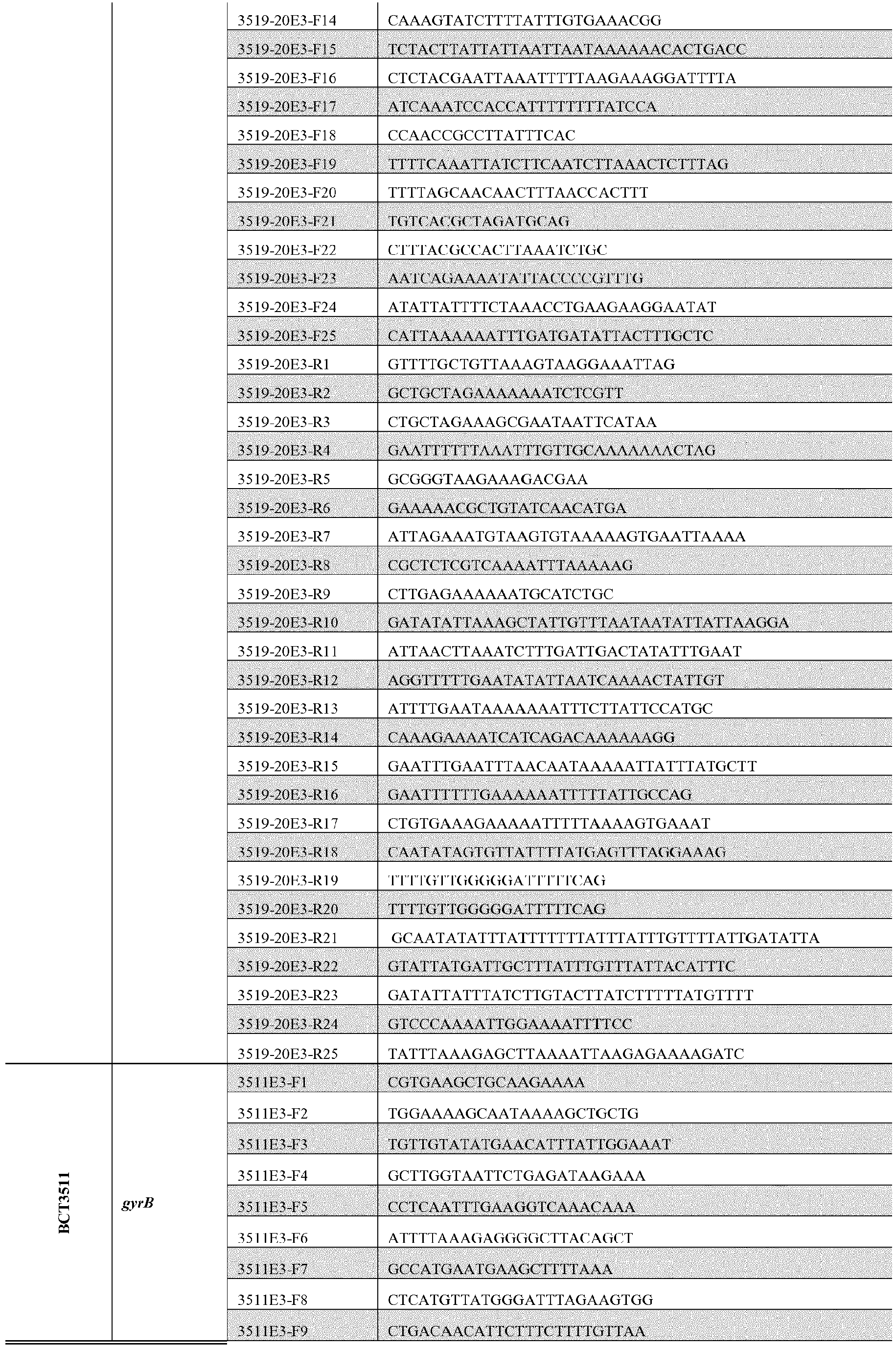

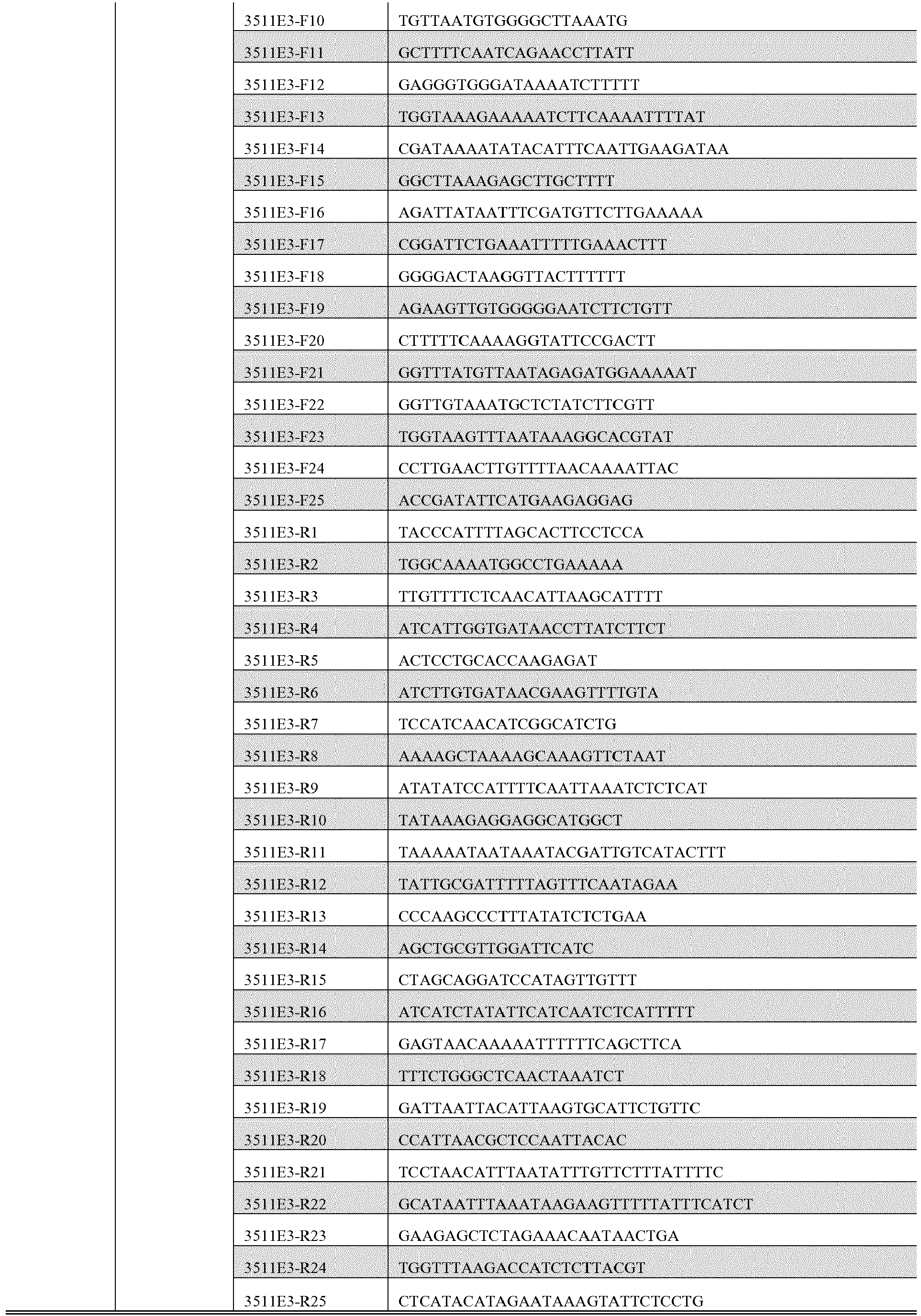

Since two of the target regions are close a single set of flanking oligos was designed to cover both targets. All primers were brought up to an initial concentration of 1mM in 10mM Tris pH 8.0 and 50uM EDTA (pH 8.0). The primers were mixed in equal proportions to create a 1mM oligo mix. Primers were designed using *B. burgdorferi* B31 genome sequence (gi|15594346) and have a GC content of 25 to 50%, spaced 6-10 nucleotides apart and have a target TM of 52°C. To ensure removal of any contaminating salts the pooled primers were dialyzed twice for 4 hours at 4°C against a 4L solution containing 10mM Tris pH 8.0 and 50uM EDTA pH 8.0 using a 5mL Float-A-Lyzer G2 (Spectrum Laboratories, Rancho Dominguez, CA) with a molecular weight cutoff of 0.5-1kDa.

The nested isothermal amplification was performed in a 225ul reaction in a 0.6mL PCR tube (Axygen Inc., Union City, California) containing 200uL of nucleic acid extract, 22.5ul Ibis 10X PCR Buffer II²¹ (Ibis Biosciences, Carlsbad, CA), 0.2uM dNTPs (Bioline, Tauton, MA), and 10uM oligo mix. The reactions were incubated at 95°C for 10min followed by a cooling to 56°C in a MJ Thermocycler (Bio-Rad Laboratories, Hercules, CA.). The reactions were then removed to a heat block at 56°C and 11.25U of BstE DNA polymerase (Lucigen, Middleton, WI) enzyme added and the reactions incubated at 56°C for 2 hours followed by an 80°C heat inactivation for 20min. The resulting reaction was used directly in the subsequent PCR without purification.

### Detection of B. burgdorferi from whole blood from patients with clinically diagnosed early Lyme disease.

Borrelia was detected by processing 2ul of *Borrelia* enriched nucleic acid extracts per PCR reaction on a previously described broad-range PCR/ESI-MS assay designed to detect and characterize *Borrelia burgdorferi* as previously described. ²¹. Electrospray ionization mass spectrometry was performed on the PLEX-ID biosensor (Abbott Molecular). Briefly, after PCR amplification, 30 µL aliquots of each PCR reaction were desalted and analyzed by mass spectrometry as previously described ²¹-²³ (herein incorporated by reference as if fully set forth herein). Analysis of amplicons from any one of the eight primer pairs in the assay can be used to positively identify *Borrelia* DNA in a specimen.

### RESULTS

The data are supportive that this new strategy can positively impact current clinical and laboratory impediments to early diagnosis of Lyme disease. A serendipitous finding of clinical importance was that the majority (∼60%), of rashes were not the classic EM in PCR positive cases.

### Detection of Lyme disease in 29 patients with classic and non-classic EM

Examination of nucleic acid extract from 1.25 ml of whole blood from the independent set of 29 endemic patients with clinically diagnosed Lyme disease with classic or non-classic EM and 44 healthy controls showed that 14 of the 29 Lyme patients, and 0 of 45 controls, (48%, p=0.0001) had detectable *B. burgdorferi.* The pre-PCR enrichment step enabled increased detection of *Borrelia* from 2 to 14 cases (7X) when assessed by the PCR/ESI-MS assay. Two-tiered serology was positive in 14 of the 29 specimens, not all of which overlapped with the 14 specimens positive by NIA/PCR/ESI-MS from Table 2. Nine of the clinically defined early Lyme disease specimens tested negative by both PCR and serology assays. However, we did not have skin isolates to determine if the *Borrelia* was actually present as a skin-restricted strain which did not disseminate to blood.^{23,24} Table 2 is presented to show PCR detection is possible even when serology is negative or not yet positive. The data support the concept that unambiguous diagnosis can be made early, before seroconversion.

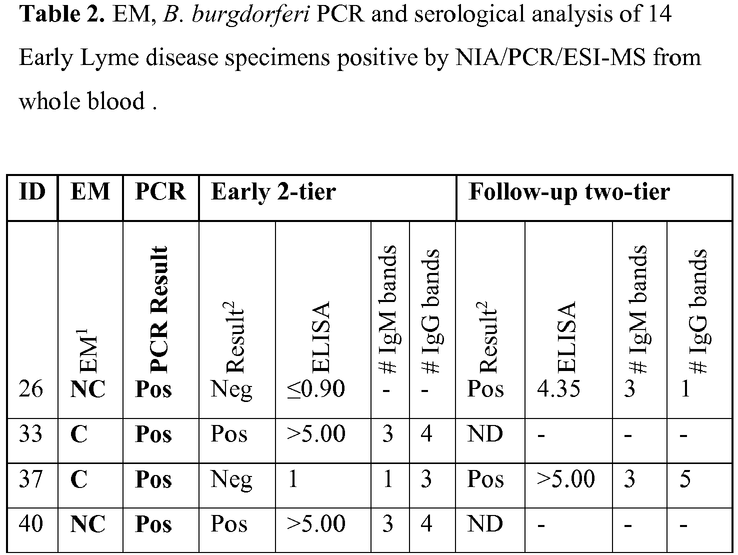

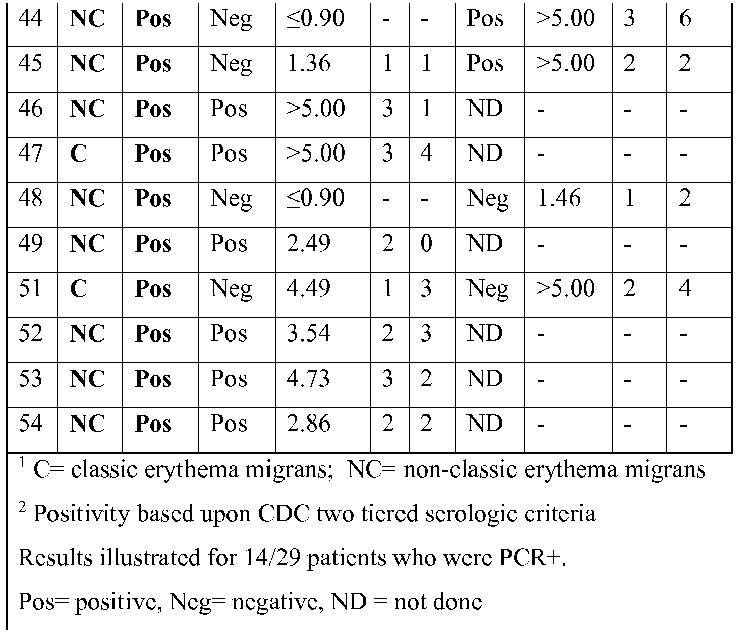

Of the 14 early Lyme specimens that tested positive by the NIA/PCR/ESI-MS assay, 6 were negative by the 2-tiered test at the time of presentation before the initiation of antibiotics (Table 2). Of these 6 specimens, 4 seroconverted by the follow-up serological testing 3 weeks later indicating that these were likely recent infections. Two of the NIA/PCR/ESI-MS positive specimens (#48 and #51) did not seroconvert sufficiently to meet the CDC surveillance criteria²⁵ but both specimens showed an increase in ELISA titers and increased IgG/IgM blot reactivity, indicating that these patients were most likely represent weak or slow responders possibly due to antibiotic therapy²⁶ or for unknown reasons. Of the 44 control patient sera, a single sample was seropositive by ELISA and IgG western blot, but was PCR negative. This low rate in our sample set from the eastern United States is consistent with the known background seroreactivity from remote exposure in an endemic region.

These results demonstrate that *B. burgdorferi* infection can be diagnosed early, and even prior to seroconversion, from whole blood in a high percentage of patients with suspected Lyme using a pre-PCR Borrelia-DNA enhancing method coupled with PCR/ESI-MS assay. Other isothermal amplification methods to generate genetic material has been described but not for Borrelia.^{7,8} In this case, a novel nested isothermal amplification (NIA) was coupled with a PCR/ESI-MS assay and results were contrasted with the two-tier serology. However, in a clinical situation, finding *Borrelia* DNA circulating in blood in an ill person with endemic area exposure will have such a strong possibility of a current infection that it would support a clinical decision to treat regardless of whether there is a classical EM, an atypical skin lesion, or extracutaneous systemic symptoms and signs suggestive of Lyme disease. The could allow for early diagnosis of the 20-30% of patients with early Lyme disease that present with "viral-like" symptoms indistinguishable for other community acquired infectious diseases. In contrast, a single serologic test, even if positive, may be difficult to interpret in a person living in an endemic area because this *single* result could represent past exposure. The present strategy has the potential to remove this ambiguity.

Work conducted during development of embodiments of the present invention led to the conclusion that hold blood may be preferable to serum or plasma for detection of Borrelia. While not limited to any mechanism, this may be due to the pathogen adhering to particular blood cells. In contrast to many diagnostics based on analysis of small (ul) of samples, the present disclosure is configured to handle larger volumes (mls), but within clinical practice volumes, to increase the detection of pathogens that may be present only in low copy numbers. A recent paper²⁷ showed that *B. burgdorferi* may be present in a high percentage of patients with Lyme disease but may require "teasing" it out with combinations of cultures and PCR. Although this method is not as easily performed within a clinically useful time period, as that which is described herein, it does suggest that there is an attainable target should it be present.

A serendipitous observation in this Example and an important clinical finding was the high percentage (57%) of PCR positive patients whose skin rash was not the classical EM but an atypical lesion (see Figure 1) that could be easily misdiagnosed. Photographs of lesions (all greater than 5 cm) were presented to four physicians experienced with Lyme disease, including two dermatologists. Without knowledge of the laboratory data they were asked to categorize the lesions into 1) those that they would expect any reasonably trained physician to recognize as EM and 2) into those that they would not fault such a physician from referring the patient or desiring supportive laboratory tests or more time for observation.^{28,29} Based on previous reports, including a vaccine trial, of atypical rashes in 25-30% of microbiologically confirmed *B. burgdorferi* infected subjects^{4,5} it was expected a similar percentage, but were surprised by almost 60%. This high percentage serves as an alert for physicians to consider Lyme disease in the differential diagnosis of patients with endemic area exposure and a rash that is not the classic EM. These atypical lesions serve as a reminder of the need for diagnostics for the endemic area patient who only has a flu-like illness during the high season for Lyme disease. Such a patient may benefit from the testing strategy described in this Example or, at the very least, closer monitoring.

It is believed that these results demonstrate a useful strategy to the diagnosis of infections when there is a paucity of pathogens or the immune response cannot provide an unambiguous result. It is likely that many of these difficult to diagnose infections will require a battery of diagnostic platforms each with advantages depending on when the patient presents.

### REFERENCES

1 Benach JL, Bosler EM, Hanrahan JP et al. Spirochetes isolated from the blood of two patients with Lyme disease. N Engl J Med 1983;308:740-2.
2 Steere AC. Lyme disease. N Engl J Med 1989;321:586-96.
3 Steere AC. Lyme disease. N Engl J Med 2001;345:115-25.
4 Steere AC, Sikand VK, Meurice F et al. Vaccination against Lyme disease with recombinant Borrelia burgdorferi outer-surface lipoprotein A with adjuvant. Lyme Disease Vaccine Study Group. N Engl J Med 1998;339:209-15.
5 Berger BW, Johnson RC, Kodner C, Coleman L. Cultivation of Borrelia burgdorferi from erythema migrans lesions and perilesional skin. J Clin Microbiol 1992;30:359-61.
6 Crowder CD, Rounds MA, Phillipson CA et al. Extraction of total nucleic acids from ticks for the detection of bacterial and viral pathogens. J Med Entomol 2010;47:89-94.
7 Mori Y, Notomi T. Loop-mediated isothermal amplification (LAMP): a rapid, accurate, and cost-effective diagnostic method for infectious diseases. J Infect Chemother 2009;15:62-9.
8 Notomi T, Okayama H, Masubuchi H et al. Loop-mediated isothermal amplification of DNA. Nucleic Acids Res 2000;28:E63.
9 Crowder CD, Matthews HE, Schutzer S et al. Genotypic variation and mixtures of Lyme Borrelia in Ixodes ticks from North America and Europe. PLoS ONE 2010;5:e10650.
10 Cox-Foster DL, Conlan S, Holmes EC et al. A metagenomic survey of microbes in honey bee colony collapse disorder. Science 2007;318:283-7.
11 Briese T, Palacios G, Kokoris M et al. Diagnostic system for rapid and sensitive differential detection of pathogens. Emerg Infect Dis 2005;11:310-3.
12 Eckburg PB, Bik EM, Bernstein CN et al. Diversity of the human intestinal microbial flora. Science 2005;308:1635-8.
13 Ecker DJ, Sampath R, Blyn LB et al. Rapid identification and strain-typing of respiratory pathogens for epidemic surveillance. Pro Natl Acad Sci U S A 2005;102:8012-7.
14 Hofstadler SA, Sampath R, Blyn LB et al. TIGER: the universal biosensor. Intl J Mass Spectrometry 2005;242:23-41.
15 Jacobs MR, Bajaksouzian S, Bonomo RA et al. Occurrence, distribution and origins of serotype 6C Streptococcus pneumoniae, a recently recognized serotype. J Clin Microbiol 2008.
16 Ecker DJ, Sampath R, Massire C et al. Ibis T5000: a universal biosensor approach for microbiology. Nat Rev Microbiol 2008;6:553-8.
17 Hannis JC, Manalili SM, Hall TA et al. High-resolution genotyping of Campylobacter species by use of PCR and high-throughput mass spectrometry. J Clin Microbiol 2008;46:1220-5.
18 Blyn LB, Hall TA, Libby B et al. Rapid detection and molecular serotyping of adenovirus by use of PCR followed by electrospray ionization mass spectrometry. J Clin Microbiol 2008;46:644-51.
19 Eshoo MW, Whitehouse CA, Zoll ST et al. Direct broad-range detection of alphaviruses in mosquito extracts. Virology 2007;368:286-95.
20 Sampath R, Russell KL, Massire C et al. Global surveillance of emerging Influenza virus genotypes by mass spectrometry. PLoS ONE 2007;2:e489.
21 Sampath R, Hall TA, Massire C et al. Rapid identification of emerging infectious agents using PCR and electrospray ionization mass spectrometry. Ann N Y Acad Sci 2007;1102:109-20.
22 Sampath R, Hofstadler SA, Blyn LB et al. Rapid identification of emerging pathogens: coronavirus. Emerg Infect Dis 2005;11:373-9.
23 Seinost G, Dykhuizen DE, Dattwyler RJ et al. Four clones of Borrelia burgdorferi sensu stricto cause invasive infection in humans. Infect Immun 1999;67:3518-24.
24 Wormser GP, Liveris D, Nowakowski J et al. Association of specific subtypes of Borrelia burgdorferi with hematogenous dissemination in early Lyme disease. J Infect Dis 1999;180:720-5.
25 CDC. Recommendations for test performance and interpretation from the Second National Conference on Serologic Diagnosis of Lyme Disease. MMWR Morb Mortal Wkly Rep 1995;44:590-1.
26 Dattwyler RJ, Volkman DJ, Luft BJ, Halperin JJ, Thomas J, Golightly MG. Seronegative Lyme disease. Dissociation of specific T- and B- lymphocyte responses to Borrelia burgdorferi. N Engl J Med 1988;319:1441-6.
27 Liveris D, Schwartz I, Bittker S et al. Improving the Yield of Blood Cultures in Early Lyme Disease. Journal of Clinical Microbiology 2011;JCM.
28 Berger BW. Erythema migrans. Clin Dermatol 1993; 11:359-62*.*
29 Berger BW. Dermatologic manifestations of Lyme disease. Rev Infect Dis 1989;11 Suppl 6:S1475-S1481.

All publications and patents mentioned in the present application are herein incorporated by reference. Various modification and variation of the described methods and compositions of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention that are obvious to those skilled in the relevant fields are intended to be within the scope of the following claims.

### EMBODIMENTS OF THE INVENTION

1. A method of nucleic acid extraction comprising:
   a) contacting a sample whole blood with beads, a proteinase, and an anionic surfactant to generate a treated sample;
   b) homogenizing said treated sample to generate a cell lysate;
   c) centrifuging said cell lysate comprising a supernatant;
   d) separating said supernatant from said cell lysate;
   e) adding magnetic particles and lysis buffer to said supernatant to generate a magnetic-particle sample, wherein said magnetic particles are configured to bind nucleic acid molecules;
   f) washing said magnetic-particle sample with a wash buffer;
   g) treating said magnetic-particle sample in order to generate a dried magnetic bead sample; and
   h) treating said dried magnetic bead sample with an elution buffer such that a purified nucleic acid sample is generated that comprises purified nucleic acid.
2. The method of Embodiment 1, further comprising subjecting said purified nucleic acid to PCR and/or isothermal nested PCR to generate amplified nucleic acid.
3. The method of Embodiment 2, further subjecting said amplified nucleic acid to mass spectrometry bioagent analysis in order to identify the source of said purified nucleic acid.
4. The method of Embodiment 3, wherein said mass spectrometry bioagent analysis comprises electrospray ionization mass spectrometry and base composition analysis.
5. A method comprising:
   a) contacting a sample comprising isolated nucleic acid with a buffer, dNTPs, and a plurality of nested PCR primer pairs configured to amplify at least part of at least one bioagent target sequence;
   b) incubating said sample with a DNA polymerase under isothermal conditions such that amplified nucleic acid is generated;
   c) inactivating said DNA polymerase; and
   d) subjecting said amplified nucleic acid to mass spectrometry bioagent analysis in order to identify the source of said isolated nucleic acid.
6. The method of Embodiment 5, wherein said mass spectrometry bioagent analysis comprises electrospray ionization mass spectrometry and base composition analysis.
7. The method of Embodiment 5, wherein said DNA polymerase comprises BstE DNA polymerase.
8. The method of Embodiment 5, wherein said incubating is conducted at about 56 degrees Celsius.
9. The method of Embodiment 5, wherein said inactivating said DNA polymerase comprises heating said sample to at least about 80 degrees Celsius.
10. The method of Embodiment 5, wherein said plurality of nested PCR primer pairs comprises at least 10 primer pairs.
11. The method of Embodiment 5, wherein said plurality of nested PCR primer pairs comprises at least 20 primer pairs.
12. The method of Embodiment 5, wherein said at least one bioagent target sequence comprises at least five bioagent target sequences.
13. The method of Embodiment 5, further comprising a step after c) but before d) of further amplifying said amplified nucleic acid without any purification of said amplified nucleic acid.

## Claims

1. A method of detecting a bioagent, comprising:
(1) a method of nucleic acid extraction comprising:
a) contacting a sample of whole blood with beads, a proteinase, and an anionic surfactant to generate a treated sample;
b) homogenizing said treated sample to generate a cell lysate;
c) centrifuging said cell lysate comprising a supernatant;
d) separating said supernatant from said cell lysate;
e) adding magnetic particles and lysis buffer to said supernatant to generate a magnetic-particle sample, wherein said magnetic particles are configured to bind nucleic acid molecules;
f) washing said magnetic-particle sample with a wash buffer;
g) treating said magnetic-particle sample in order to generate a dried magnetic bead sample; and
h) treating said dried magnetic bead sample with an elution buffer such that a purified nucleic acid sample is generated that comprises purified nucleic acid; and
(2) subjecting said purified nucleic acid to PCR and/or isothermal nested PCR to generate amplified nucleic acid.

2. The method of Claim 1, which is a method of detecting an infectious disease.

3. The method of Claim 1, further subjecting said amplified nucleic acid to mass spectrometry bioagent analysis in order to identify the source of said purified nucleic acid, wherein optionally said mass spectrometry bioagent analysis comprises electrospray ionization mass spectrometry and base composition analysis.

4. The method of claim 1, comprising:
a) contacting a sample comprising isolated nucleic acid with a buffer, dNTPs, and a plurality of nested PCR primer pairs configured to amplify at least part of at least one bioagent target sequence;
b) incubating said sample with a DNA polymerase under isothermal conditions such that amplified nucleic acid is generated;
c) inactivating said DNA polymerase; and
d) subjecting said amplified nucleic acid to mass spectrometry bioagent analysis in order to identify the source of said isolated nucleic acid,
wherein optionally said mass spectrometry bioagent analysis comprises electrospray ionization mass spectrometry and base composition analysis.

5. The method of Claim 4, wherein said DNA polymerase comprises BstE DNA polymerase.

6. The method of Claim 4, wherein said incubating is conducted at about 56 degrees Celsius.

7. The method of Claim 4, wherein said inactivating said DNA polymerase comprises heating said sample to at least about 80 degrees Celsius.

8. The method of Claim 4, wherein said plurality of nested PCR primer pairs comprises at least 10 primer pairs.

9. The method of Claim 4, wherein said plurality of nested PCR primer pairs comprises at least 20 primer pairs.

10. The method of Claim 4, wherein said at least one bioagent target sequence comprises at least five bioagent target sequences.

11. The method of Claim 4, further comprising a step after c) but before d) of further amplifying said amplified nucleic acid without any purification of said amplified nucleic acid.

12. The method of any preceding claim, wherein the sample of whole blood is 1.25mL of whole blood.

13. The method of Claim 2, which is a method of detecting Lyme disease.

14. The method of Claim 13, wherein the sample of whole blood is from a patient with skin lesions and early Lyme disease.

15. The method of Claim 13, wherein the sample of whole blood is from a patient without classic erythema migrans (EM) skin lesions.
